# EUROPEAN PATENT APPLICATION

(11) **EP 1 258 263 A1**
(43) Date of publication of application: **20.11.2002**
(21) Application number: 01112277.7
(22) Date of filing: 18.05.2001
(51) Int. Cl.: A61M 5/50, A61M 5/32

(54) **Safety device for a syringe**

(71) Applicant: Crown Cork & Seal Technologies Corporation, Alsip, IL 60803-2599 (US)
(72) Inventor: The designation of the inventor has not yet been filed
(74) Representative: Smith, Debra Jane Clare

(57) **Abstract**

A safety device 1 for a syringe having a holder 2 which can easily be gripped by a user. The holder having a flange 3, which allows the user to brace the syringe against the index and middle finger on one hand whilst depressing the plunger of the syringe. The device includes a sleeve 5, telescopically arranged inside the holder 2 and adapted to accommodate a filled syringe. The sleeve 5 held inside the holder 2 against the action of a spring 8 by means of a catch 6. On release of the catch 6, the sleeve expands rearwardly (away from the syringe needle), forming a safety enclosure into which the used syringe is drawn.

## Description

The present invention relates to a safety device for a syringe, to cover the needle after the syringe has been used and thereby to prevent needle stick injuries. In particular, the invention provides a telescopically arranged shield system, which can be activated automatically using only one hand.

US 5,697,908 describes a telescopically arranged shield system, but in this device the shield has to be manually slid into its protective, extended position by the user of the syringe. The device does not provide automatic activation of the shield and two hands are required to operate the safety system i.e. one hand to hold the syringe and one hand to slide the shield into its protective position.

EP 0 966 983 describes another safety system for a syringe, comprising a telescopically arranged shield and an automatic system for deploying the shield. Before use, the shield is held in a stowed position, alongside the barrel of a syringe holder, by stop members, which hold the shield in its stowed position against the action of a compressed spring. The spring drives the shield from its stowed to its extended, deployed position, on release of the stop members. Disengagement of the stop members is achieved by axial movement of the syringe within the syringe holder. Thus a user of the syringe depresses the syringe plunger to deliver the contents of the syringe. Once the plunger has been fully depressed and the contents of the syringe emptied, the user continues to press the plunger to move the syringe axially within the holder and thereby activate automatic release of the shield, which takes up its extended, protective position. This arrangement has the advantage that a user can automatically activate the syringe using only one hand but it has a number of disadvantages: The assembly has to be relatively large, because the shield must be of sufficient size to fully cover the needle; the release mechanism is complicated and the release mechanism can only be activated once the syringe is empty.

The aim of the present invention is to provide a safety system for a syringe, which can be activated automatically using only one hand and which is compact and simple to operate manually.

Accordingly, the present invention provides a safety device for a syringe, having an elongate barrel with a plunger extending from one end thereof and a needle extending from the opposite end. The safety device comprises a sleeve, adapted to be fitted to the barrel of the syringe; a holder, adapted to be gripped by a user when depressing the plunger, arranged surrounding the sleeve and having a catch to couple the holder and the sleeve together; and a resilient actuator arranged to move the sleeve with respect to the holder on release of the catch, from a first position in which the sleeve is stowed within the holder exposing the syringe needle, to a second position, in which the sleeve extends beyond the holder and the syringe is withdrawn within the sleeve, to fully cover the syringe needle.

Unlike the devices described in the prior art, the present invention does not have a holder coupled to the syringe and a sleeve, which is fired from the housing to cover and protect the needle. Instead, the sleeve is connected to the syringe and then, whilst the user holds the holder stationary, the sleeve is fired backwards (in the direction of the plunger) and extends to form a cocoon into which the syringe barrel and needle is withdrawn.

The shield arrangement may be adapted to expand in length, allowing the safety device to be particularly compact. For example, the shield may be provided as a number of shield segments, which are connected together and arranged telescopically one within the other. By connecting the resilient actuator to the free end of the telescopic sleeve forming the extremity of the safety cover, deployment of the resilient actuator causes all the sleeve segments to be extended one after another. Thus, the safety device may be adapted to fit a range of different size syringes and needles, merely be increasing or decreasing the number of sleeve segments.

Preferably, the catch is provided by a simple pivot arm arrangement, with a push button (or trigger) on one side of the pivot point and a retaining bar on the other side of the pivot point. When a user wishes to activate the safety shield, the push button is simply depressed, causing the retaining bar to pivot in a radial direction, thereby releasing the shield (or shield segments) which then extend to provide a safety enclosure for the syringe needle. The syringe barrel is coupled to the furthest extending part of the shield and thus, as the shield is extended, the syringe is withdrawn inside the protective enclosure, whilst the user continues to grip the holder part of the safety device.

As in conventional syringe devices, the syringe housing may be provided with a flange, to allow a user of the syringe to place their fingers in front of the flange (towards the needle end of the syringe) to resist the pressure of the syringe plunger as it is depressed. However, according to the invention, the catch trigger (which releases the safety shield to protect the needle after use) is located behind the flange (towards the plunger end of the syringe barrel). This allows the trigger to be activated by a user using only one hand without changing grip. For example, a user places two fingers in front of the flange and depresses the plunger with the thumb on the same hand, to expel the contents of the syringe. Thereafter, the user continues to hold the syringe between the fingers and merely moves the thumb to activate the trigger. Although the above construction is described with reference to a conventional flange arrangement, it should be understood that other grip arrangements are equally possible.

The safety device preferably has some form of viewing window to allow a user to check that the contents of the syringe has been fully expelled before the safety device is activated. This may be provided by an appropriately arranged aperture or apertures in the housing and/or shield or alternatively by positioning the safety device such that it covers only a part of the length of the syringe barrel and leaves the needle end thereof uncovered and visible to the user.

Advantageously, the safety device has a non-return locking arrangement to lock the shield in its extended position once it has been activated. Thus indicating that the syringe has been used and is ready for disposal. In its locked, extended position, the shield protects those handling the used syringe from needle stick injuries and also ensures that the syringe cannot be re-used.

Furthermore, the safety device according to the invention has the advantage that the shield can be shaped and tapered to fit around the syringe needle. Thus, when the safety shield is activated and the syringe withdrawn inside the cocoon formed by the sleeve, the needle is covered and the only opening into the cocoon is via a small hole (which may be only slightly larger than the thickness of the needle). Thus, this arrangement has added child safety benefits over conventional shield arrangements, where the opening at the base of the shield is generally the same or larger diameter as the syringe barrel.

The invention will now be described, by way of example only, with reference to the accompanying drawings, win which:
FIGURE 1 shows an isometric view of a safety device according to the invention, assembled on a pre-filled syringe, as supplied to the user.
FIGURE 2 shows a side section view of the syringe and safety device shown in Figure 1.
FIGURE 3 shows an isometric view of the safety device shown in Figures 1 and 2, with the tapered end of the syringe inserted into a needle and the syringe ready for use.
FIGURE 4 shows a side section view of the syringe and safety device shown in Figures 3, with the syringe plunger fully depressed (i.e. in the position where the syringe has been used).
FIGURE 5 shows an isometric view of the safety device shown in Figures 1 to 4, with the safety sleeve fully extended to form an enclosure and the syringe withdrawn inside the enclosure.
FIGURE 6 shows a side section view of the syringe and safety device with the safety sleeve activated, as shown in Figure 5.

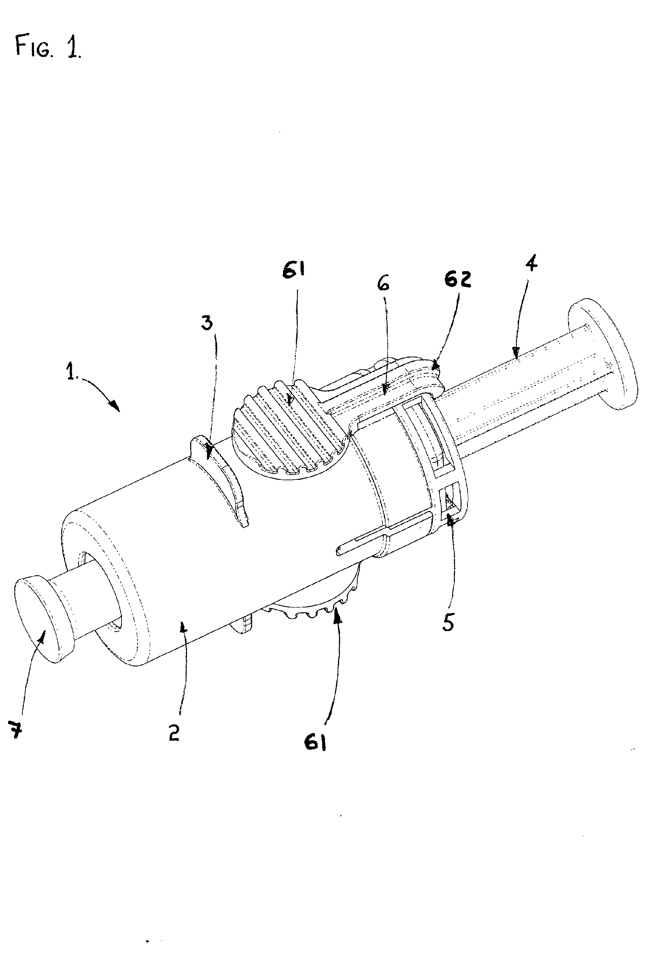

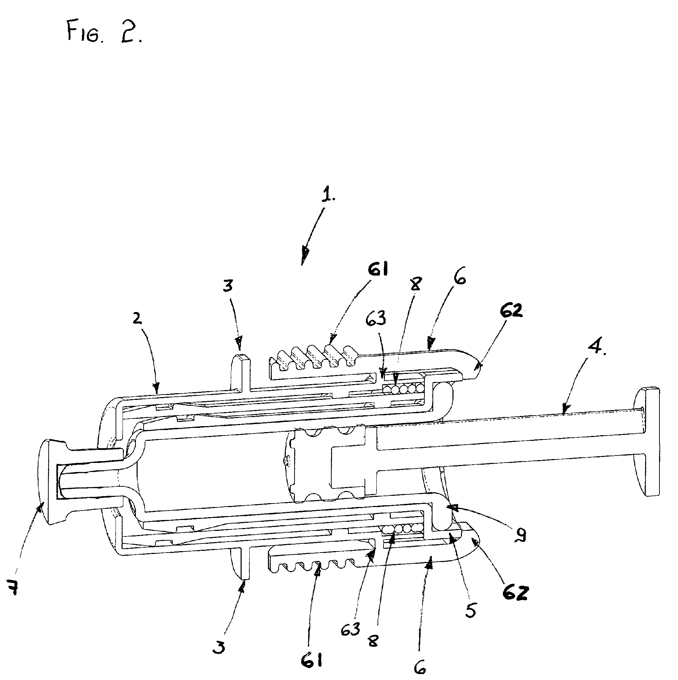

Referring to Figure 1, a safety device 1 for a syringe comprises a holder 2, having a flange 3 by which the user of a syringe may hold the syringe barrel against the force applied to depress the plunger 4. The safety device 1 further comprises a sleeve 5, which is adapted to be fitted to the syringe barrel. The sleeve 5 is held in its stowed position (within the holder 2), by means of a catch 6, pivotally connected to the holder 2. The catch 6 comprises a push tab 61 at one end and a hook 62 at the other end. The hook 62 is arranged to overlie the end of the sleeve 5, thereby coupling the sleeve 5 and holder 2 together (as shown more clearly in Figure 2). Finally, a dust cover 7 may be provided to cover and seal the open end of the syringe barrel.

The construction of the safety device 1 and syringe can be more clearly understood with reference to Figure 2. The syringe barrel 9 is held in the sleeve 5 The sleeve 5 is then held in its stowed position inside the holder 2 against the action of a compressed biasing means (such as a spring 8). A hook 62 on the catch overlies the end of the sleeve 5, thereby coupling it to the holder 2. When a user wishes to release the catch 6, they simply press on the push button 61, pivoting the catch lever arm about its connection 63 to the holder 2, thereby pivoting the hook 62 radially clear of the sleeve 5 and allowing the sleeve 5 to activate.

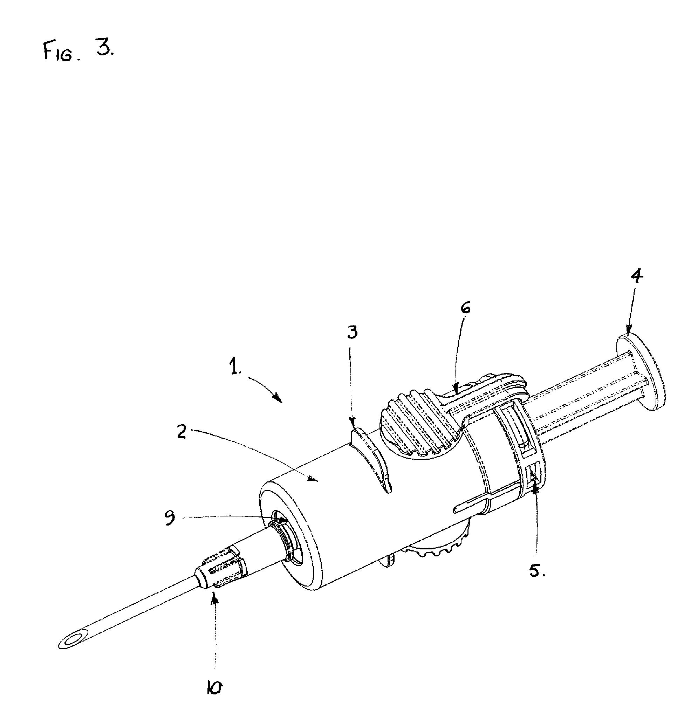

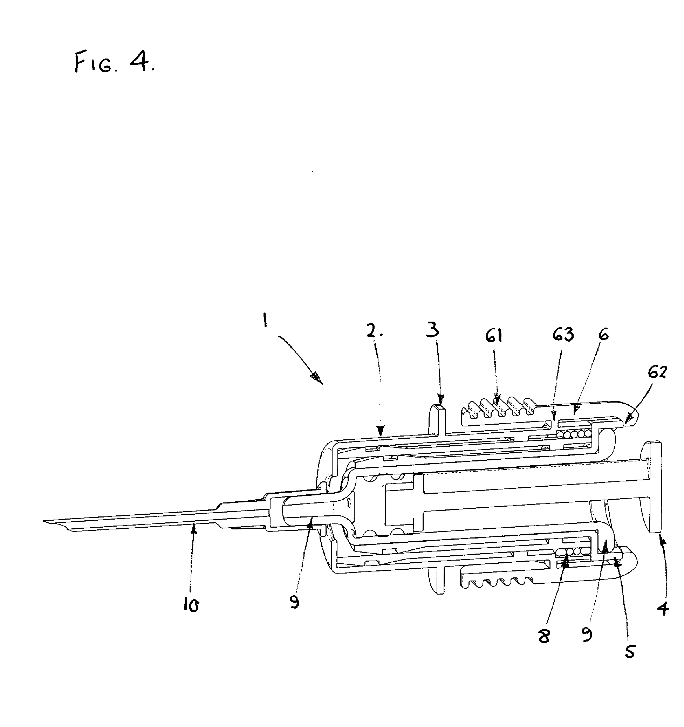

Figure 3, shows the syringe and safety device of figures 1 and 2 ready to use. The dust cover 7 has been removed from the open, tapered end of the syringe barrel 9 and replaced with a needle 10. Referring to Figure 4, the user then injects the contents of the syringe by depressing the plunger 4. The user will normally support the syringe with the index finger and middle finger of one hand placed either side of the holder 2 against the flange 3 and will then depress the plunger 4 using the thumb on the same hand. From figure 4, it will be appreciated that the arrangement of the push buttons 61 behind the flange 3 (i.e. towards the plunger end of the syringe) makes the safety device particularly easy to activate with one hand. Once the user has fully depressed the plunger 4, they simply move their thumb and third finger to press on the push buttons 61. Two push buttons are preferably provided to allow the safety device to be operated easily regardless of whether the user is left or right handed. Furthermore two buttons 180 degrees apart ensures that the sleeve 5 is held evenly against the force of the spring 8. Alternatively, only one button 61 may be provided, which can then be simply actuated by movement of the user's thumb from the plunger 4 of the syringe to the button 61.

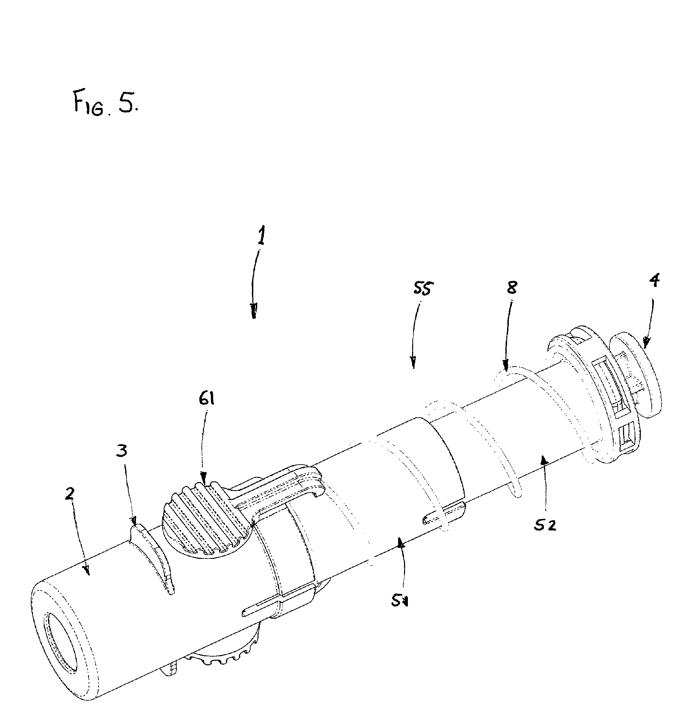

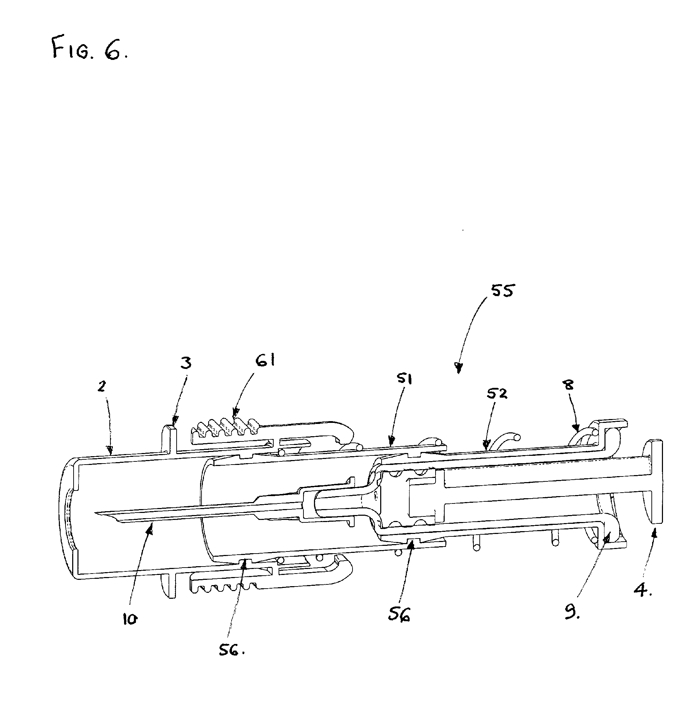

Referring to figures 5 and 6, once the user has used the syringe and depressed the buttons 61, the sleeve 5 is released and expands under the action of the spring 8 forming a safe enclosure 55 for the syringe. Because the syringe is held by the sleeve 5, instead of the holder 2, rather than the sleeve forming an enclosure around the needle, the sleeve 5 forms and enclosure 55, into which the syringe is drawn (as shown in Figure 6). In a particularly advantageous embodiment of the invention, the sleeve 5 may be provided by a plurality of telescopically arranged sleeves 51 52. This arrangement allows the safety device to be particularly compact. The sleeve segments 51 and 52 are provided with latches 56 which restrict the expansion of the arrangement at the end of travel, thereby preventing the sleeve segments 51, 52 and holder 2 becoming disassociated. The latches 56 are preferably provided by a bead 57, which engages in a slot 58. The bead 57 and slot 58 are designed to lock the sleeve or sleeves (5, 51, 52) in their expanded configuration once the push buttons 61 have been actuated, Thus the activated safety device provides an enclosure around the used syringe, preventing needle stick injuries and preventing re-use of the syringe.

Although the embodiment shown in the drawings comprises a sleeve composed of two telescopically arranged segments it will be appreciated that the sleeve may comprise a single segment, where the size of the device is not an issue. Furthermore, although the sleeve is shown coupled to the top of the syringe barrel, adjacent to the plunger, it will be appreciated that a single sleeve may be provided coupled towards the needle end of the syringe barrel, to provide a compact arrangement using only a single segment sleeve, provided the syringe barrel is sufficiently short, to allow the device to be operated easily by a user.

## Claims

1. A safety device 1 for a syringe, having an elongate barrel 9 with a plunger 4 extending from the one end of the barrel and a needle 10 extending from the opposite end of the barrel, the safety device comprising:
a sleeve 5 adapted to be fitted to the barrel of the syringe,
a holder 2 adapted to be gripped by a user, arranged surrounding the sleeve and having a catch 6 to couple the holder 2 and the sleeve 5 together, and
a resilient actuator 8 arranged to move the sleeve 5 with respect to the holder 2 on release of the catch 6, from a first position in which the sleeve 5 is stowed within the holder 2 exposing the syringe needle 10, to a second position, in which the sleeve 5 extends beyond the holder and syringe is withdrawn within the sleeve 5, to fully cover the syringe needle 10.

2. A safety device according to claim 1, wherein the sleeve 5 is adapted to expand in length.

3. A safety device according to claim 2, wherein the sleeve 5 comprises a plurality of sleeve sections 51, 52 connected together and arranged telescopically with respect to one another.

4. A safety device according to any of the preceding claims, wherein the catch 6 comprises a pivot arm, the arm having
a trigger 61, adapted to be pressed by a user and
a retainer 62, arranged to couple the sleeve 5 and holder 2 together,
the trigger 61 and retainer 62 arranged on either side of a pivot point 63, such that when a user applies pressure to the trigger, the arm pivots about the pivot point, disengaging the coupling between the sleeve and the holder and thereby releasing the sleeve.

5. A safety device according to claim 4, wherein the catch 6 comprises two pivot arms radially arranged at 180 degrees to one another.

6. A safety device according to any one of the preceding claims, wherein the resilient actuator comprises a compressed spring 8, arranged to drive the sleeve to its extended position on release of the catch.

7. A safety device according to any one of the preceding claims, wherein a grip portion 3 is provided on the housing and the catch trigger 61 is located behind the grip portion 3, towards the plunger end of the syringe, so that a user of the syringe can depress the plunger and then activate the trigger without changing grip.

8. A safety device according to claim 7, wherein the grip portion comprises a flange 3 extending substantially perpendicular to the main axis of the syringe barrel 9 and arranged to allow a user to support the syringe whilst depressing the plunger 4 thereof.

9. A safety device according to any one of the preceding claims, wherein the safety device is adapted to allow the user of a syringe, to which the safety device is attached, to view the contents of the syringe, to ensure that all the contents is expelled prior to activation of the safety device.

10. A syringe having an elongate barrel, with a plunger moveable within the barrel and extending from one end thereof and a needle extending from the opposite end of the barrel, the syringe further comprising a safety device according to any one of the preceding claims.
